# EUROPEAN PATENT APPLICATION

(11) **EP 3 708 223 A1**
(43) Date of publication of application: **16.09.2020**
(21) Application number: 19162796.7
(22) Date of filing: 14.03.2019
(51) Int. Cl.: A61N 5/06, B60Q 3/70, B60J 3/02, B60Q 3/252, B60N 2/879

(54) **VEHICLE COMPRISING A LIGHT SOURCE ARRANGEMENT THAT IS DESIGNED TO EMIT ELECTROMAGNETIC RADIATION INTO A PASSENGER CABIN OF THE VEHICLE AND CORRESPONDING LIGHT SOURCE ARRANGEMENT**

(71) Applicant: AUDI AG, 85045 Ingolstadt (DE); LG Electronics Inc., Yoido-dong Seoul 150-721 (KR)
(72) Inventor: RITTGER, Dr. Lena, 85051 Ingolstadt (DE); MERTENS, Joris, 85051 Ingolstadt (DE); OH, Jiwon, Gangseo-gu Seoul (KR); DIBOINE, Romain, Seocho-gu Seoul (KR); SONG, Jieun, Songpa-gu Seoul (KR)
(74) Representative: Hofstetter, Schurack & Partner

(57) **Abstract**

The invention is concerned with a vehicle (10) comprising a light source arrangement (16) that is designed to emit electromagnetic radiation (21) into a passenger cabin (11) of the vehicle (10). The invention is characterized in that the light source arrangement (16) is designed to generate, as the electromagnetic radiation (21), a cosmetic light (22) that invokes a predefined biological reaction of human skin.

## Description

The invention is concerned with a vehicle comprising a light source arrangement that is designed to emit electromagnetic radiation into a passenger cabin of the vehicle. In contrast to a normal light source arrangement that provides light in the visible range for enhancing the visibility of objects in the passenger cabin, the light source arrangement of the inventive vehicle is provided for invoking a biological reaction that is different from human vision. The invention also comprises a corresponding light source arrangement that may be installed in the vehicle.

Document DE 10 2015 209 603 A1 describes a vehicle that provides a light source arrangement for a so-called light therapy, that is, for providing a type of light that suppresses the production of the hormone melatonin in a human body of a user of the vehicle. In general, a light therapy may be achieved by providing visible light with an extremely high intensity, that is, with more than 5,000 Ix or even more than 10,000 Ix.

Document DE 10 2016 205 309 A1 describes a vehicle with a light source arrangement for emitting infrared light that is directed to a face of a driver in order to support an infrared camera in taking clear pictures. Such an infrared light is of the so-called near infrared spectrum that allows the infrared camera to generate camera images of the face of the person even in the dark. The person will not sense this infrared light as it is neither visible to the person nor will the person feel any warmth.

Document DE 10 2013 006 793 A1 describes a vehicle with windows that provide a dimming function for attenuating light entering the passenger cabin through the windows.

It is an object of the present invention to invoke or invigorate a biological function of a person sitting in a passenger cabin of a vehicle.

The object is achieved by the subject matter of the independent claims. Advantageous further embodiments of the invention are described in the dependent claims, the following description and the drawings.

The invention provides a vehicle comprising a light source arrangement that is designed to emit electromagnetic radiation into a passenger cabin of the vehicle. In other words, the biological function of the person is invoked on the basis of electromagnetic radiation. To this end, the light source arrangement is designed to generate, as the electromagnetic radiation, a so-called cosmetic light that invokes a biological reaction of human skin. In other words, the electromagnetic radiation emitted by the light source arrangement is provided for the human skin rather than for the human eyes. To this end, a spectrum of the electromagnetic radiation is adapted in such a way that a cosmetic light functionality is provided. Note that the effect of a biological reaction of the human skin is not a mere byproduct, but rather at least 30%, preferably 40% of the emitted electromagnetic energy consists of electromagnetic radiation that invokes a specific biological reaction of human skin. Known cosmetic light types are, for example, ultraviolet light (e.g. for the production of vitamin D and/or for tanning) and infrared light in the so-called medium infrared spectrum or MIR-spectrum (for warming up the skin).

The invention provides the advantage that by the light source arrangement the vehicle is enhanced by a cosmetic functionality regarding the skin of at least one person using the vehicle.

The invention also comprises embodiments which provide additional advantages.

In one embodiment, the cosmetic light comprises UV-light (UV - ultraviolet) for invoking a tanning of human skin. To this end, the electromagnetic radiation may comprise light with a wavelength in the range of 280 nm to 380 nm (measured in air at a pressure of 1 bar and a temperature of 20°C). Providing UV-light yields the advantage that a person may obtain a suntan while using the vehicle.

In one embodiment, the cosmetic light comprises infrared light in the MIR-spectrum, i.e. light with a wavelength larger than 1 µm, but preferably shorter than 100 µm, especially in a range of 3 µm to 50 µm (measured in air at 1 bar and 20°C). The infrared light in the MIR-spectrum is for heating up the skin and thus increasing a blood flow in the skin. In other words, the electromagnetic radiation makes the person feel warm at the skin that receives the electromagnetic radiation. This provides the advantage that due to the increased blood flow an increase in blood oxygen is available for the cells of skin.

In one embodiment, at least one respective light source of the light source arrangement is arranged in a sun visor of the vehicle, i.e. in a sun visor over a windscreen of the vehicle or over a side window of the window. As a light source, a unit comprising at least one LED (light emitting diode) may be provided. Providing the light source in a sun visor yields the advantage that, as the sun visor may be extended or unfolded, the light source may be moved closer to the skin of a face of a person.

Additionally or alternatively, at least one respective light source of the light source arrangement may be arranged in a head rest of a vehicle seat of the vehicle. A person sitting behind that vehicle seat, e.g. in a second row of vehicle seats, may thus face the light source in the head rest of the vehicle seat in front. In other words, the light source may be arranged at a back side of the head rest. This provides the advantage that a person sitting right behind the vehicle seat directly faces the light source such that the electromagnetic radiation may reach the skin of the face of that person.

Additionally or alternatively, at least one respective light source of the light source arrangement may be arranged in a ceiling of the passenger cabin. This provides the advantage that the light source will not obstruct a movement of a head of a person.

In one embodiment, at least one light source of the light source arrangement is designed as or arranged in a detachable or removable accessory for placing on a face of a user. In other words, the user may remove or detach the accessory from a holding or mount and may place the accessory directly on the face. This provides the advantage that the user may hold the head in a position that is independent of the holding or mount as at least one light source as provided in the accessory and may be arranged directly on the face. There is no need for the user to hold the head in a specific position for receiving the electromagnetic radiation.

In one embodiment, said accessory is a flexible and/or foldable mask. The accessory will therefore adapt to a shape of the face. A flexible mask may be provided in the form of a flexible electric circuitry, as it may be provided on the basis of wires to which the at least on light source may be connected. A foldable mask may be provided in the form of several printed circuit boards (PCBs) which may be interconnected on the basis of hinges.

In one embodiment, the mask comprises at least one window and/or through hole for the eyes of the user. In other words, the user may view the surroundings in the passenger cabin while wearing the mask. This provides the advantage that the user may still operate at least one vehicle component, for example an infotainment system, and/or may read a text and/or watch a movie.

In one embodiment, the mask comprises a head strap for holding the mask to a head of the user. In other words, the mask will hold to the head without any manual support by the user. This provides the advantage that the user has the hands free for other tasks.

In one embodiment, the detachable accessory, e.g. the mask, comprises a battery and a charging unit and is designed to operate wirelessly in the detached state. In other words, while the accessory is detached from its mount or holding, the energy for operating or running the at least one light source of the accessory is provided from within the accessory, i.e. from its battery. This provides the advantage that no cable is needed that might get entangled with another object in the passenger cabin. A charging unit for charging a battery of the accessory may be based on induction charging and/or galvanic electric contacts. As an alternative, the accessory may be connected to an electric voltage source of the vehicle by means of a cable. This provides the advantage that the accessory may be used for a longer time than would be the case with a battery-driven accessory.

The inventive vehicle is preferably provided as a motor vehicle, preferably a passenger vehicle or a truck, or as a bus.

For providing or building a vehicle of the described type, the invention also provides a light source arrangement for a vehicle, wherein the light source arrangement comprises at least one light source. The light source arrangement is designed as a sun visor or a head rest. In other words, for installing the light source arrangement, the sun visor or head rest needs to be installed in the vehicle. This automatically provides the light source arrangement in a passenger cabin of the vehicle. The at least on light source of the light source arrangement is designed to emit UV-light for invoking a tanning of human skin and/or infrared light in the MIR-spectrum for heating up the skin and thus increasing a blood flow in the skin.

The invention also comprises additional embodiments of the light source arrangement that comprise features which have already been described in connection with the embodiments of the vehicle. The corresponding embodiments of the light source arrangement are therefore not described here again.

The invention also comprises the combinations of the features of the different embodiments.

In the following an exemplary implementation of the invention is described. The figures show:
- Fig. 1: a schematic illustration of a perspective view into a passenger cabin of an embodiment of the inventive vehicle; and
- Fig. 2: a schematic illustration of a perspective view of a detachable accessory provided in the vehicle.

The embodiment explained in the following is a preferred embodiment of the invention. However, in the embodiment, the described components of the embodiment each represent individual features of the invention which are to be considered independently of each other and which each develop the invention also independently of each other and thereby are also to be regarded as a component of the invention in individual manner or in another than the shown combination. Furthermore, the described embodiment can also be supplemented by further features of the invention already described.

In the figures identical reference signs indicate elements that provide the same function.

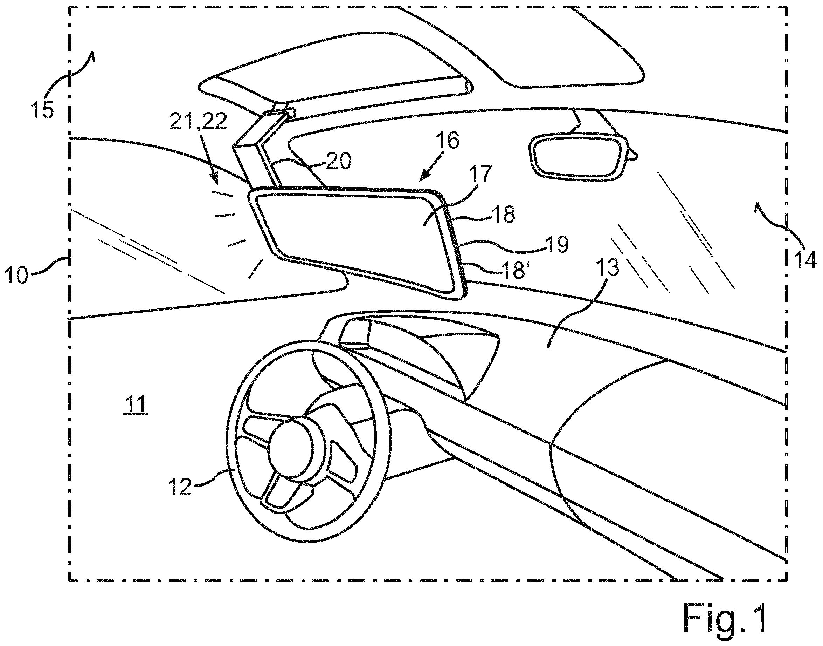

Fig. 1 shows a perspective view into a vehicle 10. What is shown is a passenger cabin 11 from a viewpoint of a passenger seat. For orientation purposes, Fig. 1 shows a steering wheel 12, a dashboard 13, a windscreen 14 and a ceiling 15. The vehicle 10 can e.g. be a passenger vehicle or a truck or a bus.

In the vehicle 10 a light source arrangement 16 with at least one light source 17 may be provided. The embodiment shown in Fig. 1 illustrates that at least one light source 17 may be arranged in a sun visor 18 which may be a sun visor 18 for the windscreen 14 or for a side window (not shown).

The at least one light source 17 may be designed to emit electromagnetic radiation 21 which have the effect of a cosmetic light 22 on human skin, especially on the skin of a face of a human being. In particular, the cosmetic light 22 may consist of or comprise UV-light and/or infrared light in the MIR-spectrum. Thus, while using the vehicle 10, a person sitting in front of the accessory 18' may be able to receive the cosmetic light 22 with the skin of the face. Thus, a tanning and/or an increase in blood flow in the skin may be gained.

The at least one light source 17 of the light source arrangement 16 may be arranged in a detachable accessory 18'. In the example shown, the sun visor 18 may be detachable from a holding or mount 20 which may support the sun visor 18 at the ceiling 15. For making the accessory 18' detachable, a mechanism as it is available in the prior art may be used.

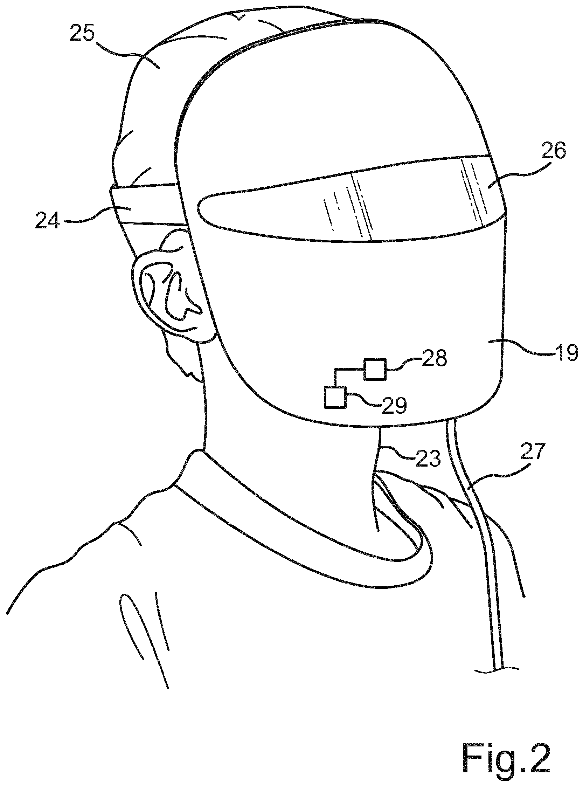

Fig. 2 illustrates how the accessory 18', may be designed as a flexible mask 19, i.e. a mask 19 that may be changeable in shape. To this end, the mask 19 may provide a flexible body and/or hinges. Fig. 2 also illustrates how a person or user 23 may lay the mask 19 on the face and adapt the shape of the mask 19 to the face by rolling or folding the mask 19 around the face. The mask 19 may be fixed by means of a head strap 24 around a head 25 of the user 23. The mask 19 may provide at least one through hole or window 26 such that a person may see peek through the mask 19 while wearing the mask 19.

For providing the light source 17 with energy, a cable 27 may be provided which may connect the light source 17 inside the mask 19 with an electric source of the vehicle 10, e.g. the central battery of the vehicle 10. Alternatively, the mask 19 or in general the detachable accessory 18' may comprise its own battery 28 and a charging unit 29 for which a corresponding charger may be provided in the vehicle 10, for example in the mount 20.

Overall, a cosmetic light 22 may therefore be provided in the passenger cabin 11 of the vehicle 10. To this end, a light source arrangement 16 for UV-light and/or infrared light may be provided in the passenger cabin 11. This can be done in the form of a sun visor which may be unfolded for obtaining a tanning and/or warming functionality. The light source arrangement may be provided as a detachable mask 19, for example in the form of the sun visor 18, or at a head rest or at the ceiling 15. Such a mask 19 may be detached by a user and worn directly on the face for receiving UV-light and/or infrared light with the skin of the face.

Thus, a cosmetic or wellness-related application may be provided to a user of the vehicle 10 while the vehicle 10 is driving. A driver may use this functionality in an autonomous driving mode of the vehicle 10. A passenger of the vehicle 10 may use the functionality any time. Thus, the time in the vehicle 10 may be used effectively and/or in a relaxing manner.

A light source 17 may be provided at the inner side of a sun visor, i.e. the side of the sun visor 18 facing the passenger cabin 11 and not facing the windscreen 14. A user may activate the functionality of a cosmetic light when the sun visor 18 is unfolded in front of the window of the windscreen 14 or a side window. The electric connection for the light source may be based on a cable 27 as it may also already be used for the lights for a mirror. The electric cable may be provided in a foldable mount 20 which holds the sun visor 18.

A light source may be provided in a flexible mask which may be part of the vehicle. After detaching the mask or taking the mask out of a pocket, the user may wear the mask and activate the at least one light source of the mask for receiving the cosmetic light. Energy for the mask may be provided via a battery 28 which may be recharged while the mask is attached to an electric source of the vehicle, e.g. while in the pocket or case or while attached to the mount 20. Alternatively, the cable 27 may be provided.

Other ways of implementing the light source arrangement are also possible. For example, at least one UV-light source and/or at least one infrared-light sources may be provided in the ceiling 15 and/or in a head rest of the vehicle. From there, they may also emit the cosmetic light to the facial skin of at least one user of the vehicle.

Overall, the example shows how a cosmetic light is provided in a vehicle by the invention.

## Claims

1. Vehicle (10) comprising a light source arrangement (16) that is designed to emit electromagnetic radiation (21) into a passenger cabin (11) of the vehicle (10),
**characterized in that**
the light source arrangement (16) is designed to generate, as the electromagnetic radiation (21), a cosmetic light (22) that invokes a predefined biological reaction of human skin.

2. Vehicle (10) according to claim 1, wherein the cosmetic light (22) comprises UV-light for invoking a tanning of the human skin.

3. Vehicle (10) according to any of the preceding claims, wherein the cosmetic light (22) comprises infrared light in the MIR-spectrum for heating up the skin and thus increasing a blood flow in the skin.

4. Vehicle (10) according to any of the preceding claims, wherein at least one respective light source (17) of the light source arrangement (16) is arranged in a sun visor (18) of the vehicle (10) and/or in a head rest of a vehicle seat of the vehicle and/or in a ceiling (15) of the passenger cabin (11).

5. Vehicle (10) according to any of the preceding claims, wherein at least one light source (17) of the light source arrangement (16) is arranged in a detachable accessory (18') for placing on a face of a user (23).

6. Vehicle (10) according to claim 5, wherein the accessory (18') is a flexible and/or foldable mask (19).

7. Vehicle (10) according to claim 6, wherein the mask (19) comprises at least one window (26) and/or though hole for the eyes of the user (23).

8. Vehicle (10) according to claim 6 or 7, wherein the mask (19) comprises a head strap (24) for holding the mask (19) to a head (25) of the user (23).

9. Vehicle (10) according to any of claims 5 to 8, wherein the accessory (18') comprises a battery (28) and a charging unit (29) and is designed to operate wirelessly in the detached state or wherein the accessory (18') is connected to an electric voltage source of the vehicle (10) by means of a cable (27).

10. Light source arrangement (16) for a vehicle (10), the light source arrangement (16) comprising at least one light source (17), wherein the light source arrangement (16) is designed as a sun visor (18) or a head rest of a vehicle seat,
**characterized in that**
the at least one light source (17) is designed to emit UV-light for invoking a tanning of human skin and/or infrared light in the MIR-spectrum for heating up the skin and thus increasing a blood flow in the skin.
